# EUROPÄISCHE PATENTSCHRIFT

(11) **EP 1 062 237 B1**
(45) Veröffentlichungstag und Bekanntmachung des Hinweises auf die Patenterteilung: **28.06.2006**
(21) Anmeldenummer: 98961234.6
(22) Anmeldetag: 30.11.1998
(51) Int. Cl.: C07K 14/47, C07K 16/18, A61K 38/17

(54) **METALLHALTIGE RIBONUKLEOTIDPOLYPEPTIDE**
RIBONUCLEOTIDE POLYPEPTIDES CONTAINING METAL
POLYPEPTIDES RIBONUCLEOTIDIQUES CONTENANT DU METAL

(30) Priorität: 13.03.1998 DE 19811047
(43) Veröffentlichungstag der Anmeldung: 27.12.2000
(73) Patentinhaber: Fraunhofer-Gesellschaft zur Förderung der angewandten Forschung e.V., 80686 München (DE)
(72) Erfinder: KIESEWETTER, Stefan, D-73760 Ostfildern (DE); KUHN, Eckehard, D-72636 Frickenhausen (DE); KOCH-PELSTER, Brigitte, D-71522 Backnang (DE); BRUNNER, Herwig, D-70569 Stuttgart (DE)
(86) Internationale Anmeldenummer: PCT/EP1998/007722
(87) Internationale Veröffentlichungsnummer: WO 1999/047561

(56) Entgegenhaltungen:
- WO-A-97/04007
- DE-A- 19 628 895
- J H WISSLER ET AL.: "An endogenous bioactive metallo-ribonucleo-polypeptide: a copper-containing monocytic blood vessel morphogen as a novel type of "wound-hormone"" BIOCHEM. ENG. [INT. CONGR.], MEETING DATE 1986. EDITORS H CHMIEL, W P HAMMES AND J P BAILEY ,1987, Seiten 385-391, XP000614120 Fischer, Stuttgart, BRD
- CHEMICAL ABSTRACTS, vol. 106, no. 3, 19. Januar 1987 (1987-01-19) Columbus, Ohio, US; abstract no. 16813, J H WISSLER ET AL.: "Structure and function of a monocytic blood vessel morphogen (angiotropin) for angiogenesis in vivo and in vitro; a copper-containing metallo-polyribonucleo-polypeptide as a novel and unique type of monokine " XP002022899 & PROTIDES BIOL. FLUIDS, Bd. 34, 1986, Seiten 525-536,
- E C DELL'ANGELICA ET AL.: "Primary structure and binding properties of calgranulin C, a novel S100-like calcium-binding protein from pig granulocytes" JOURNAL OF BIOLOGICAL CHEMISTRY, Bd. 269, Nr. 46, 18. November 1994 (1994-11-18), Seiten 28929-28936, XP002022898 AMERICAN SOCIETY OF BIOLOGICAL CHEMISTS, BALTIMORE, MD., US in der Anmeldung erwähnt

## Beschreibung

Die vorliegende Erfindung betrifft metallhaltige Ribonukleotidpolypeptide (RNP) sowie Verfahren zu deren Herstellung, deren Verwendung und Arzneimittel, die Ribonukleotidpolypeptide oder deren molekularbiologische Äquivalenzstrukturen und/oder Teile und/oder Derivate enthalten.

Die Gewebehomeostase des Körpers, seiner Organe und Gewebe ist abhängig von Regulationsmechanismen der Angiogenese. Diese beeinflusst sowohl die Gewebereparatur und Wundheilung, Gewebeneubildung in der Embryogenese und den Reproduktionszyklen als auch das Anwachsen, die Rückbildung und die Zerstörung von Tumoren, Transplantationen und gefäßversorgten und gefäßfreien Geweben.

Die WO-A-9704007 offenbart spezifische bioaktive metallhaltige Ribonukleotid-Polypeptide (RNP), wobei als Metall vorzugsweise Calcium, Kupfer oder Zink vorkommt.

Aufgabe der vorliegenden Erfindung ist es, weitere metallhaltige Ribonukleotid-Polypeptide als nichtmitogene Mediatoren der Gewebehomeostase bereitzustellen, mit denen vorrangig die Gewebereparatur, Wundheilung Angiogenese und Neovaskularisierung beeinflusst werden kann. Aufgabe der Erfindung ist weiter die Bereitstellung eines Verfahrens zur Herstellung dieser nichtmitogenen Mediatoren sowie eines Arzneimittels, das diese nichtmitogenen Mediatoren enthält.

Gelöst werden diese Aufgaben durch die Gegenstände der Patentansprüche 1 bis 3.

Von den Erfindern wurde gefunden, daß es nichtmitogene zelluläre Mediatoren auf Nukleinsäurebasis mit definierter Sequenz gibt, die spezifisch die Bildung von Blutgefäßen in vivo und in vitro verursachen können und biologisch spezifische, natürlich wirkende nichtmitogene Mediatoren der Angiogenese bzw. des Richtungswachstums von Blutgefäßsprossen darstellen.

Die von den Erfindern nachgewiesene neue Klasse zellulärer Morphogene für Endothelzellen liegt in Form eines bioaktiven Metall-Ribonukleotidpeptids (RNP) vor. Diese wird Angiotropin genannt.

Die Struktur von Angiotropin kann den Ribonukleotidproteinen (RNP) zugeordnet werden. Es besteht aus einen Protein-Teil (ARP = Angiotropin Related Protein) und einem RNA-Teil (ARNA = Angiotropin-RNA). Für die Bildung des Komplexes aus ARP und ARNA ist Cu(II) essentiell. Neben dem Kupferion enthält Angiotropin ein Ca(II)-Ion. Für die vielfältigen biologischen und biochemischen Funktionen des Angiotropins sind weiter Mg(II)-Ionen nützlich.

Der Protein-Teil (ARP) besteht aus einem Protein, das der Familie der S100-Proteine (Dell'Angelica et al., J. of Biological Chemistry, Vol. 269, No. 46, S. 28929-28936 (1994)) zugeordnet werden kann und vorzugsweise 91 Aminosäuren lang ist. Die Primärstruktur dieses bevorzugten ARP ist wie folgt:

Ca2+
NH2-TKLEDHLEGIINIFHQYSVRLGHYDTLIKRELKQLITKELPNTLKNT
Ca2+ Zn2+
KDQGTIDKIFQNLDANQDEQVSFKEFVVLVTDVLITAHDNIHKE-COOH

Es weist zwei EF-Hand Motive auf und darüber hinaus eine Bindungsstelle für Zink(II)-Ionen. Die Dissoziationskonstanten K_{D} der Metallionenkomplexe betragen für Ca(II)-Ionen 10,0 µM bzw. 0,1 µM für Zink(II)-Ionen und 1,0 µM für Kupfer(II)-Ionen.

Erfindungsgemäß sind die verwendbaren ARNAs weiter wie folgt definiert:
(a1) ARNA I 
Klon-3a (ARNA I)
AAAAAAAAGGUUUUCAUGCGUGCUCACAGAUCAGCUCUUUCUGGAUUGAAAAGCU
AAGCACAGAACAUGGAAAAUUCCUUUCAUAUGGCUGUGUUUACAAACAAAAAGU
AUAAACAUCUUGAGCAAACAGAAAUGGUUGAGGAAAACUUUGUUAGCAGAUUAG
 oder
(a2) ARNA VI

Klon P-10 (ARNA VI)
UUACAGCUCUUCUGUUUAUAAGUUAUUCAAUACCAAAUUAGUAGUUUGUAUGUUA
UAAAUUUGUAGGAAAAUAAUUAUAUAUGCUUACUUUGUACAUAAAAAUAAAAACAU
GACUUCUUUAGACACUCCUUCAUUAGAAAUAAAAUAAAAUAAACUAUUAGCAGUUU
GACUUCAUGUUCUGUCUGUAGGUCAUGGAAUCCUGUCCUUACAAUAUUUAUUGAU
UGUGAAAAUAUCAGUAAAUAAGCAAUUGAAUAUGUUUACCUUUUCUUCUAGUCAC
UAUGUUCUUAGAGUUAUGACA

Die erfindungsgemäßen RNPs sind durch die folgenden Eigenschaften gekennzeichnet:
- zellselektive morphogene Wirkung in vitro auf isolierte, primäre und/oder klonierte Blutkapillarendothelzellen in Kultur zur nichtmitogenen Induktion der Änderung des Zellphänotyps aus dem konfluenten Zustand, zur nichtmitogenen Änderung der spatiotemporalen suprazellulären organisation der Zellen zu dreidimensionalen organoiden, kapillarähnlichen Strukturen in Kultur;
- spezifische chemotropische Wirkung auf Blutgefäße in vivo,
- Induktion eines Richtungswachstums von Blutgefäßen in vivo,
- Induktion einer Neovaskularisierung von Geweben durch gerichtetes Einwachsen von Blutgefäßen

Bei den erfindungsgemäßen RNPs ist der Proteinteil durch Wechselwirkungen an den RNA-Teil gebunden.

Gegenstand der Erfindung ist auch ein Verfahren zur Herstellung und Gewinnung der bioaktiven RNPs, dadurch gekennzeichnet, daß man die Zellen, z.B. Leukozyten oder Entzündungsgewebe homogenisiert oder Leukozyten kultiviert und die entstandenen RNPs aus den Homogenaten oder aus den Überständen der Kulturlösung durch Standardmethoden gewinnt. Beispielsweise kann aus den Überständen serumfreier Massenzellkulturen Concanavalin-A aktivierter Schweineblut-Leukozyten und Leukozyten aus ischämischen/infarktösen Herzmuskelzellen Angiotropin isoliert und zur Homogenität aufgereinigt werden.

Das Verfahren zur Herstellung und Gewinnung der bioaktiven RNP-Morphogene der Zellen bzw. Leukozyten und des Entzündungsgewebes ist dadurch gekennzeichnet ist, daß man Zellen bzw. Leukozyten des retikulo-endothelialen Systems der Leukozyten und des Entzündungsgewebes, kultiviert und die entstandenen RNP-Morphogene aus den Homogenaten oder aus der überstehenden Kulturlösung gewinnt.

Grundsätzlich ist es auch möglich, die Zellen, z. B. Leukozyten auf Mediatoren direkt ohne Kultur aufzuarbeiten.

Die Kultur der Zellen (Leukozyten) kann grundsätzlich in jedem die Zellen (Leukozyten) erhaltenen Medium durchgeführt werden.

Zur Kultur von Zellen, wie Leukozyten, wird den Kulturmedien bei einer geplanten Dauer der Kultur über 1 Stunde meist Serum, beispielsweise Kalbsserum oder Pferdeserum, zugesetzt, da die Serumbestandteile für die Erhaltung der Lebensfunktionen der Zellen günstig sind. Wenn jedoch die serumhaltige Kulturlösung auf Proteine (Mediatoren) aufgearbeitet werden soll, die durch die Kultur erzeugt werden, bereitet die Gewinnung der meist nur in geringen Konzentrationen enthaltenen Produktproteine wegen der Vielzahl der aus dem Serum stammenden fremden Proteine große Schwierigkeiten. Außerdem kann dabei nicht mit Sicherheit festgestellt werden, ob ein bestimmter Mediator humoraler oder zellulären Ursprungs ist und von welcher Spezies er stammt; d.h. ob er ein Mediator der Spezies ist, deren Zellen kultiviert wurden, oder der Spezies, von der das verwendete (meist heterologe) Serum stammt.

Das erfindungsgemäß bevorzugt verwendete vollsynthetische Zellkulturmedium enthält die üblichen Stoffgruppen, wie Salze, Zucker, Aminosäure, Nukleoside und Nukleosidbasen, Vitamine, Vitaminoide, Coenzyme und Steroide in wäßriger Lösung. Es ist dadurch gekennzeichnet, daß es zusätzlich einen oder ein Gemisch von mehreren Stoffen enthält, die sich als besonders wertvoll für die Lebensfähigkeit und das Wachstum der Leukozyten und ihre Fähigkeit zur Mediatorproduktion erweisen. Zu diesen Stoffen gehören ungesättigte Fettsäuren, Flavonoide, Ubichinone, Vitamin C und Mevalolacton.

Das Zellkulturmedium wird zur längerdauernden Zell- oder Leukozytenkultur vorzugsweise ohne Zusatz von Serum verwendet. Statt dessen erhält es mindestens ein definiertes Protein, das in einer besonders bevorzugten Ausführungsform hochreines, molekular einheitliches Serumalbumin ist.

In weiteren bevorzugten Ausführungsformen kann das erfindungsgemäß verwendete, vollsynthetische serumfreie Zellkulturmedium noch weitere, für die Kultur von Leukozyten günstige Verbindungen aus den Stoffklassen der Polyhydroxyverbindungen und Zucker, Aminosäuren, Nukleoside, anionischen Verbindungen und/oder Vitamine, deren Verwendung in den bekannten Kulturmedien nicht üblich ist, enthalten. Die Bestandteile des erfindungsgemäß verwendeten Mediums sind in ihren Mengenverhältnissen so aufeinander abgestellt, daß die Konzentration der Komponenten im Medium weitgehend den natürlichen Konzentrationsbereichen des Plasmas angepaßt sind; vgl. Ciby-Geigy AG (Herausgeber) (1969) in Documenta Geigy, Wissenschaftliche Tabellen, 7. Auflage Geigy S.A., Basel.

Vorzugsweise ist das Zellkulturmedium frei von Tensiden, Schwermetallsalzen und Farbstoffen, die Zellen schädigen und die Gewinnung der gewünschten Zellprodukte aus der Kulturlösung stören können.

Besonders bevorzugt ist für die Kultur der Leukozyten im Verfahren der Erfindung des Zellkulturmedium mit der in nachstehender Tabelle 1 angegebenen Zusammensetzung.

Zur Herstellung des Mediums wird Wasser mit der ASTM-1-Qualität verwendet; vgl. ASTM D-1193-70 Standard-Specification for Reagent Water 1970; Annual Book of ASTM-Standards, Easton Maryland, ASTM 1970. Es ist darüber hinaus von möglichen Endotoxin-Kontaminationen durch Ultrafiltration an tensidfreien Membranen mit der Ausschlussgrenze von 10000 Daltons befreit. Das fertige Medium wird filtersterilisiert an tensidfreien Membranen mit ≤ 0,2 µm Porengröße.

**Tabelle 1**

| Nr. | Komponente | mol/l |
|---|---|---|
| 1 | KCl | 5,0 m |
| 2 | KH₂PO₄ | 0,2 m |
| 3 | NaCl | 120,0 m |
| 4 | Na₂HPO₄ | 0,8 m |
| 5 | Na₂SO₄ | 0,8 m |
| 6 | L-Ascorbinsäure | 0,2 m |
| 7 | Cholinchlorid | 50,0 µ |
| 8 | 2-Desoxy-D-ribose | 5,0 µ |
| 9 | D-Galactose | 0,5 m |
| 10 | D-Glucose | 5,0 m |
| 11 | D-Glucurono-γ-lacton | 0,1 m |
| 12 | Glycerin | 50,0 µ |
| 13 | myo-Inosit | 0,5 m |
| 14 | Na-Acetat | 0,2 m |
| 15 | Na-Citrat | 50,0 *µ* |
| 16 | Na-Pyruvat | 0,1 m |
| 17 | D-Ribose | 20,0 µ |
| 18 | Bernsteinsäure | 0,1 m |
| 19 | Xylit | 10,0 *µ* |
| 20 | D-Xylose | 20,0 *µ* |
| 21 | CaCl₂ | 2,0 m |
| 22 | MgCl₂ | 1,0 m |
| 23 | NaHCO₃ | 10,0 m |
| 24 | Humanes Serumalbumin | 7,7 *µ* |
| 25 | Penicilin | 1,0 *µ* |
| 26 | Streptomycin | 2,0 *µ* |
| 27 | L-Glutamin | 1,0 m |
| 28 | L-Alanin | 0,2 m |
| 29 | L-Asparagin | 0,1 m |
| 30 | L-Asparaginsäure | 0,1 m |
| 31 | L-Glutaminsäure | 0,1 m |
| 32 | Glycin | 0,2 m |
| 33 | L-Prolin | 0,1 m |
| 34 | 2L-Serin | 0,1 m |
| 35 | L-Arginin | 0,1 m |
| 36 | 4-Aminobenzoesäure | 2,0 *µ* |
| 37 | L-Cystein | 0,2 m |
| 38 | L-Histidin | 0,1 m |
| 39 | L-Hydroxyprolin | 10,0 *µ* |
| 40 | L-Isoleucin | 0,2 m |
| 41 | L-Leucin | 0,2 m |
| 42 | L-Lysin-HCl | 0,2 m |
| 43 | L-Methionin | 0,1 m |
| 44 | L-Ornithin | 50,0 *µ* |
| 45 | L-Phenylalanin | 0,1 m |
| 46 | Sarcosin | 50,0 µ |
| 47 | Taurin | 0,1 m |
| 48 | L-Threonin | 0,2 m |
| 49 | L-Trypthophan | 50,0 µ |
| 50 | L-Tyrosin | 0,1 m |
| 51 | 1-Valin | 0,2 m |
| 52 | Glutathion reduziert | 3,0 µ |
| 53 | Carnosin | 5,0 µ |
| 54 | Mevalolacton | 5,0 µ |
| 55 | Adenin | 50,0 µ |
| 56 | Adenosin | 50,0 µ |
| 57 | Citidin | 50,0 µ |
| 58 | Guanin | 5,0 µ |
| 59 | Guanosin | 20,5 µ |
| 60 | Hypoxanthin | 5,0 µ |
| 61 | 5-Methylcytosin | 5,0 µ |
| 62 | Thymidin | 20,0 µ |
| 63 | Thymin | 5,0 µ |
| 64 | Uracil | 5,0 µ |
| 65 | Uridin | 20,0 µ |
| 66 | Xanthin | 5,0 µ |
| 67 | Biotin | 1,0 µ |
| 68 | C-Ca-pantothenat | 5,0 |
| 69 | Ergocalciferol | 0,5 µ |
| 70 | D, L-Carnitin | 50,0 µ |
| 71 | Folsäure | 5,0 µ |
| 72 | D, L-α-Liponsäure | 2,0 µ |
| 73 | Menadion | 0,2 µ |
| 74 | Nicotinsäureamid | 20,0 µ |
| 75 | Pyridoxal-Hcl | 5,0 µ |
| 76 | Pyridoxin-Hcl | 2,0 µ |
| 77 | Riboflavin | 1,0 µ |
| 78 | Rutin | 5,0 µ |
| 79 | Thiamin-Hcl | 5,0 µ |
| 80 | D, L-α-Tocopherylacetat | 1,0 µ |
| 81 | Vitamin-A-acetat | 1,0 µ |
| 82 | Vitamin K | 0,2 *µ* |
| 83 | Vitamin B¹ | 0,5 *µ* |
| 84 | Vitamin U¹² | 1,0 *µ* |
| 85 | Cholesterin | 1,0 *µ* |
| 86 | Coenzym-Q₁₀ | 0,1 *µ* |
| 87 | Linolsäure | 1,0 *µ* |
| 88 | Linolensäure | 5,0 *µ* |
| 89 | Ölsäure | 5,0 *µ* |
| 90 | Äthanol | 1,0 m |
| 91 | Ph 7,10 | -- |
| 92 | Concanavalin A | 50,0 n |

Je nach der Art der gewünschten Produkte werden entweder gemischte Zell- oder Leukozytenpopulationen oder einzelne Zell- oder Leukozytenarten kultiviert. Die Herstellung und Kultur der Zellen bzw. Leukozyten muß unter sterilen Bedingungen erfolgen. Die Kultur wird ausreichend lange Zeit durchgeführt, um eine befriedigende Mediatorausbeute zu erhalten. Hierfür hat sich eine Dauer von etwa 10 bis 50 Stunden als geeignet erwiesen. Bei kürzeren Zeiten ist die Mediatorausbeute zu gering, so daß das Verfahren unwirtschaftlich ist. Bei einer Kulturdauer über etwa 50 Stunden ist andererseits das Medium erschöpft und die Zellen beginnen abzusterben, so daß eine Erhöhung der Ausbeute nicht mehr zu erwarten ist.

Die Kultur der Zellen bzw. Leukozyten wird bei einer Temperatur von etwa 30 bis 42 °C, vorzugsweise von etwa 37 °C durchgeführt. Bei niedrigeren Temperaturen ist der Kulturprozeß unbefriedigend, während bei Temperaturen über 42 °C die Leukozyten geschädigt werden.

Die Kultur wird mit einer Konzentration von etwa 10⁶ bis 5 x 10⁸ Zellen/ml, vorzugsweise bis zu 10⁷ bis 10⁸ Zellen/ml durchgeführt. Bei niedrigeren Zellkonzentrationen ist die Ausbeute pro Volumeneinheit der Kulturlösung zu gering. Das Verfahren wird infolge zu großer Kulturvolumina unwirtschaftlich. Bei Zellkonzentration über 5 x 10⁸ Zellen/ml tritt sehr rasch Verarmung des Mediums an Nahrungsstoffen auf.

Die Kultur kann an der Atmosphäre durchgeführt werden. Vorzugsweise wird über die Kultur ein erhöhter Kohlendioxidparitaldruck aufrecht erhalten, der bis etwa 10 Vol%, insbesondere bis etwa 2 Vol% reichen kann. Von großer Bedeutung ist die Sauerstoffversorgung der Kultur. Sie kann beispielsweise durch Einleiten von Luft sichergestellt werden. Um eine Kontaminierung der Kultur zu vermeiden, ist die zugeführte Luft vorzugsweise sterilisiert und hitzedekontaminiert, d.h. von Endotoxinen und anderen organischen Bestandteilen befreit. Die Lösung kann während der Kultur gerührt oder geschüttelt werden. Als Zellstimulanz wird vorzugsweise Con A eingesetzt.

Zur Beendigung der Kultur werden die Zellen bzw. Leukozyten von der Kulturlösung abzentrifugiert, die anschließend auf die entstandenen Angiotropine aufgearbeitet wird. Um eine Schädigung der Zellen und damit eine Verunreinigung der Kulturlösung durch Zellbestandteile zu vermeiden, wird die Kultur bei verhältnismäßig niedriger Beschleunigung, d.h. etwa 300 bis 400 x g, zentrifugiert. Nach dem Abtrennen des Großteils der Zellen vom Überstand wird dieser zweckmäßigerweise bei höherer Beschleunigung erneut zentrifugiert, um restliche Schwebeteilchen zu entfernen. Die abgetrennten Leukozyten können entweder erneut kultiviert, kryopräserviert oder einer anderen Verwendung zugeführt werden.

Außer durch Kultur von Leukozyten können die bioaktiven RNP-Morphogene der Erfindung auch aus Entzündungsgewebe gewonnen werden. Dort entstehen sie durch die Ansammlung der Leukozyten infolge des durch die Gewebeschädigung ausgelösten Entzündungsprozesses. Das Entzündungsgewebe kann in üblicher Weise gewonnen und für die Präparation der RNP verwendet werden. Dazu wird das Entzündungsgewebe in Pufferlösung homogenisiert und die löslichen Bestandteile (Exsudat) werden von den unlöslichen Strukturbestandteilen des Gewebes getrennt.

Vorzugsweise wird entzündetes infarziertes Herzmuskelgewebe verwendet, welches durch Ligation des linken vorderen absteigenden Astes der linken Koronararterie mittels einer transfemoralen Kathedertechnik während 24 Stunden gebildet wird. Der Leukozyten enthaltende, entzündete Herzmuskelteil wird bei 0 bis 4 °C von nicht infarziertem, gesunden Gewebe abgetrennt.

Für die Isolierung und Gewinnung der bioaktiven RNP der Erfindung ist die Aufarbeitung eines sehr großen Kulturlösungsvolumens erforderlich. Zu Beginn des Reinigungsverfahrens ist es deshalb aus praktischen Gründen notwendig, eine möglichst effektive Verminderung des zu behandelnden Volumens durchzuführen. Die Kulturlösung enthält neben den geringen Mengen an erzeugten Substanzen, darunter hauptsächlich Proteine, das Gemisch der Bestandteile des Mediums. Vorteilhafterweise wird deshalb im ersten Schritt der Reinigung eine Abtrennung der entstandenen Proteine von den Bestandteilen des Mediums und gleichzeitig von dem großen Volumen der wäßrigen Lösung durchgeführt. Dies kann durch eine selektive Aussalzung der Proteine aus der Kulturlösung bewirkt werden, die beispielsweise durch Zugabe eines sulfates oder Phosphates erreicht wird. Nachstehend wird die Fällung der Proteine am Beispiel der Aussalzung durch Zugabe von Ammoniumsulfat zu der Kulturlösung beschrieben.

Durch Sättigung der Kulturlösung mit Ammoniumsulfat wird der Großteil der entstandenen Proteine zusammen mit gegebenenfalls enthaltenen Serumalbumin ausgefällt. Nach dem Abtrennen des Substanzniederschlags, beispielsweise durch Zentrifugieren, kann dieser in der nachstehend beschriebenen Weise in seine einzelnen Komponenten aufgetrennt und die enthaltenen bioaktiven RNP gewonnen werden. Der erhaltene Überstand enthält neben den löslichen Bestandteilen des Mediums auch den in gesättigter Ammoniumsulfatlösung löslichen Teil der Substanzen, unter denen sich auch ein Teil der bioaktiven RNP befindet. Der Überstand wird konzentriert und die enthaltenen Substanzen werden daraus in der nachstehenden Weise gewonnen. Wenn die proteinhaltige Kulturlösung mit Ammoniumsulfat bis zur Sättigung versetzt wird, fällt der größere Teil der begleitenden Proteine aus. Auf diese Weise wird ein Proteingemisch erhalten, das aus einer Anzahl verschiedener Proteine besteht und dessen Trennung in die einzelnen Komponenten infolgedessen mühsam ist. In einer bevorzugten Ausführungsform des Verfahrens der Erfindung wird das in der Kulturlösung enthaltene Proteingemisch deshalb bereits in der Fällungsstufe in mehrere Fraktionen aufgetrennt. Diese Auftrennung in mehrere Proteinfraktionen ist möglich, da die einzelnen Proteine bei verschiedenen Ammoniumsulfatkonzentrationen ausfgefällt werden. Vorzugsweise wird die Kulturlösung im Verfahren der Erfindung deshalb stufenweise mit Ammoniumsulfat bis zu bestimmten Sättigungsgraden versetzt, wobei in jeder Fraktion der Teil der Proteine ausfällt, dessen Löslichkeitsprodukt unterhalb des jeweiligen Sättigungsgrades liegt. Durch geeignete Wahl der Sättigungsgrenzen der einzelnen Fraktionen kann im Verfahren der Erfindung eine grobe Auftrennung in Gruppen von Proteinen bereits bei der Fällung erzielt werden.

Beispielsweise wird die Kulturlösung zunächst bis zu einer Sättigung von 35 % mit Ammoniumsulfat versetzt. Der erhaltene Proteinniederschlag wird abgetrennt. Danach wird der Sättigungsgrad der überstehenden Lösung auf 45 % erhöht. Es bildet sich erneut ein Proteinniederschlag, der abgetrennt wird. Anschließend wird die überstehende Lösung auf einen Sättigungsgrad von 90 % eingestellt. Der dabei erhaltene Proteinniederschlag wird ebenfalls abgetrennt. Die überstehende Lösung dieser Fällung wird beispielsweise durch EntwässerungsDialyse oder Ultrafiltration konzentriert.

Die Salzfällung der Proteine wird ebenso wie die nachfolgende Reinigung vorzugsweise bei einer Temperatur von etwa 0 bis 10 °C, insbesondere etwa 0 bis 4 °C durchgeführt. Die für die Reinigung verwendeten Lösungen besitzen einen ph-Wert zwischen 5 und 9, insbesondere zwischen 6 und 8. Um eine Ph-Konstanz der Lösung zu erreichen, wird vor der Salzfällung vorzugsweise ein starker Puffer, z.B. 0,1 mol/l Phosphatpuffer, zugesetzt. Zur Aufrechterhaltung des Redoxpotentials der Proteine wird den Lösungen vorzugsweise cystein in einer Menge von 0,001 mol/l zugesetzt. Sterile Bedingungen für die Proteinreinigung sind nicht erforderlich.

Die bei der Salzfällung erhaltenen Proteine können nach Auflösen in einem Proteine nicht schädigenden Medium direkt der nachstehend beschriebenen Reinigung und Auftrennung zugeführt werden. Der Überstand der letzten Fällungsstufe wird konzentriert, beispielsweise durch Entwässerungsdialyse oder Ultrafiltration. Dabei werden alle Verbindungen mit einem Molekulargewicht von größer als etwa 300 bis 500 Daltons, d.h. auch die Proteine und Peptide dieser Fraktion, als Retentat quantitativ erhalten.

Die in der vorstehend beschriebenen Stufe erhaltenen Proteinfraktionen enthalten die bioaktiven RNP der Erfindung im Gemisch mit zahlreichen Fremdproteinen (andere sezernierte Proteine gegebenenfalls Serumalbumin und gegebenenfalls CON). Die Fremdproteine liegen in den Gemischen in weit überwiegender Menge vor. Durch eine Reihe von Reinigungsschritten müssen die bioaktiven RNP angereichert und von den Fremdproteinen soweit befreit werden, daß diese ihre molekulare biologische Spezifität nicht mehr stören. Die bioaktiven RNP selbst sind ebenfalls eine Stoffklasse, die in ihre einzelnen, spezifisch wirkenden Individuen aufgetrennt wird.

Allgemein bestehen Reinigungsverfahren für Eiweißkörper (Proteine) und andere Naturstoffe aus einer Sequenz kombinierter Trennungsverfahren, welche Molekülgrößen-, Ladungs-, Form-, Strukturstabilitäts- und Moleküloberflächenbeschaffenheitsunterschiede zwischen dem gesuchten Wirkstoff und den begleitenden Fremdstoffen zur Trennung ausnutzen. Dementsprechend können zahlreiche Kombinationen verschiedenster Trennungsverfahren zur Reinigung eines Proteins erarbeitet werden. Für die Handhabungseigenschaften, technische Ausführbarkeit, Automatisierbarkeit und Wirtschaftlichkeit eines Reinigungsverfahrens sowie für die Qualität des gesuchten Naturproduktes ist deshalb nicht allein die Art der verwendeten Trennungsschritte von Bedeutung, sondern insbesondere deren optimierte Gestaltung und deren sinnvolle Kombination in einer Reinigungssequenz innerhalb des Rahmens der Strukturstabilität und der anderen Strukturparameter des gesuchten Wirkstoffes. Das heißt auch, daß selbst die Benutzung gleicher oder ähnlicher Trennungsprinzipien (z.B. Molekularsiebfiltration, Dialyse, Ionenaustauschadsorption, usw.), aber in unterschiedlicher Kombination, für die Handhabungsfähigkeit und Wirtschaftlichkeit des Reinigungsverfahrens entscheidend sein können. In bestimmten Fällen ist die Benutzung oder Unterlassung einer einzigen Technik (z.B. Hydroxylapatitchromatographie, Zonenpräzipitationschromatographie, usw.) an einer bestimmten Stelle der Reinigungssequenz, oder innerhalb einer begrenzten Teilsequenz, von ausschlaggebender Bedeutung für die Qualität des gesuchten Wirkstoffes sowie für die Handhabungsfähigkeit und die Wirtschaftlichkeit seines Reinigungsverfahrens. Klar aufgezeigt werden diese allgemeinen Zusammenhänge und Grundprinzipien der Naturstoffreinigung beispielsweise an der allgemein bekannten Tatsache, daß in einem wirtschaftlich vernünftigen und technisch handhabungsfähigen Naturstoffreinigungsverfahren ein säulenchromatographischer Reinigungsschritt oder ein Lyophilisierungsschritt nicht sinnvoll ist, bevor das Gesamtausgangsvolumen oder die Ausgangskonzentration der begleitenden Fremdbestandteile des Wirkstoffrohextraktes nicht auf mindestens 1/500 bis 1/1000 durch andere Verfahrensschritte reduziert wurde.

Für die Reinigung der einzelnen Proteinfraktionen bieten sich eine Mehrzahl von an sich einzeln in der Biochemie bekannten Reinigungsschritten an. Beispiele für solche Reinigungsschritte sind: Präparative und analytische Molekularsiebfiltration, Anionen- und Kationenaustascherchromatographie bzw. Eintopfadsorptionsverfahren, Chromatographie an Hydroxylapatit, Zonenpräzipitationschromatographie und Kreislauf- oder Kaskadenmolekularsiebfiltration.

Bereits durch einmalige Durchführung eines der genannten Reinigungsverfahren kann eine beträchtliche Menge an Begleitproteinen von den bioaktiven RNP abgetrennt werden. Jedoch halten die in den Fraktionen enthaltenen Substanzen trotz ihres verschiedenen Molekulargewichts häufig sehr stark aneinander. Sie werden beispielsweise in der Molekularsiebfiltration durch das Bestehen nicht idealer Gleichgewichte bei Proteinpolyelektrolyten oft unvollständig entsprechend ihrem Molekulargewicht getrennt. Es empfiehlt sich deshalb, mindestens zwei der genannten Trennverfahren hintereinander durchzuführen. Vorzugsweise werden die bioaktiven RNP-Aktivität enthaltenden Proteinfraktionen mindestens drei der genannten Reinigungsschritte nacheinander unterzogen.

Alle Kombinationen der erwähnten Trennschritte sind Gegenstand des erfindungsgemäßen Verfahrens. Dabei gehört es zum Kenntnisstand des Fachmanns, daß bestimmte Folgen von Trennschritten weniger sinnvoll sind als andere Kombinationen. Beispielsweise ist dem Fachmann bewußt, daß bei Durchführung einer präparativen Molekularsiebfiltration nach einer analytischen Molekularsiebfiltration neben der unhandlichen Verfahrensweise auch ein schlechteres Gesamtergebnis in Bezug auf die Trennwirkung erhalten wird als bei umgekehrter Reihenfolge.

Die Molekularsiebfiltration bewirkt eine Auftrennung der Proteine entsprechend ihrem Molekulargewicht. Da ein überwiegender Teil der begleitenden Fremdproteine ein anderes Molekulargewicht als die bioaktiven RNP aufweist, kann ihre Abtrennung auf diese Weise erreicht werden. Für die Trennung der Substanzen nach ihrem Molekulargewicht wird ein hydrophiles, in Wasser quellendes Molekularsieb verwendet. Beispiele für geeignete Molekularsiebe sind mit Epichlorhydrin vernetzte Dextrane (Sephadex), mit Acrylamid vernetzte Agarosen (Ultrogele) und raumvernetzte Acrylamide (Biogele), deren Auschlußgrenzen größer als die zur Auftrennung verwendeten Separationsgrenzen sind.

Die Molekularsiebfiltration wird, falls mehrerer Trennstufen zur Anwendung kommen, vorzugsweise als einer der Ersten durchgeführt. Je nach dem Längen-Durchmesser-Verhältnis der verwendeten Säule und dem Teilchendurchmesser der Gelmatrix, welche die theoretische Bodenzahl der Säule beeinflussen, wird die Molekularsiebfiltration als "präparativ" oder "analytisch" bezeichnet. Sie wird als "präparativ" bezeichnet, wenn die Chromatographie an Säulen mit einem Dimensionsverhältnis Länge: Durchmesser bis 10:1 und einer Beladung von bis zu 1/3 des Säuleninhalts bzw. unter voller Ausnutzung des gesamten, matrixzentypischen separationsvolumens durchgeführt wird. "Analytisch" bedeutet ein Längen-Durchmesser-Verhältnis über 10:1, vorzugsweise etwa 50:1, und eine Beladung bis maximal 3 % des Säuleninhaltes, auch bei HPLC Versionen.

Bei der präparativen Molekularsiebchromatographie werden Gelmatrizen mit möglichst großer Teilchengröße benutzt, um schnelle Durchflußraten der oft etwas viskosen Proteinlösungen bei möglichst niedrigen Drucken zu erzielen. Bei der analytischen Molekularsiebfiltration wird die Teilchengröße der Gelmatrix so klein wie möglich gewählt, um eine maximale theoretische Bodenzahl der Säule bei technisch und sicherheitsmäßig verwertbarem Druck und einer Flußgeschwindigkeit der mobilen Phase von 2 bis 4 cm/h zu erreichen. Diese Parameter sind von der Gelmatrixstruktur abhängig und von Gel zu Gel verschieden.

Falls mehrere präparative Molekularsiebfiltrationen nacheinander durchgeführt werden, kann die Separationsgrenze abgestuft gewählt werden. Daran anschließend kann eine analytische Molekularsiebfiltration mit entsprechend abgestuften Separationsgrenzen durchgeführt werden. Die Ausschlußgrenze des verwendeten Gels muß jedenfalls größer als etwa 10000 Daltons sein, um eine Volumenverteilung der Angiotropine zwischen der stationären Gelmatrixphase und der mobilen wässrigen Pufferphase zu ermöglichen.

Die "Ausschlussgrenze" bezeichnet den hydrodynamischen Parameter eines gelösten Teilchens, welcher der Porengröße der Gelmatrix entspricht. Teilchen mit größerem hydrodynamischen Parameter können nicht mehr in die Gelmatrix eindringen (Volumenverteilungskoeffizient K_{D} = 0). Die "Separationsgrenze" bezeichnet einen zur Trennung von gelösten Teilchen zweckmäßigerweise festgesetzten hydrodynamischen Paramter, der zwischen einem Volumenverteilungskoeffizienten K_{D} = 0 und K_{D} = 1 liegt.

Zur Molekularsiebfiltration werden die Substanzen gelöst in einer die Substanzen nicht schädigenden Flüssigkeit auf das Molekularsieb aufgebracht. Ein spezielleres Beispiel für ein geeignetes Lösungsmittel ist 0,003 mol/l Natriumkaliumphosphatlösung mit einem Gehalt von 0,3 mol/l NaCl und 0,001 mol/l Cystein und einem ph-Wert von 7,4. Nach der Filtration werden die RNP enthaltenden Fraktionen in der nachstehend beschriebenen Weise konzentriert und gegebenenfalls einem weiteren Reinigungsschritt unterzogen.

Die Anionenaustauscher für die Reinigung der Substanzen eignen sich beispielsweise mit Epichlorhydrin vernetzte Dextran-(Sephadex) oder Zellulosematrizen, an welche funktionelle Gruppen mit Anionenaustauscherkapazität gekoppelt sind. Sie können nach Verwendung durch Regeneration wiederholt gebraucht werden. Bevorzugt wird ein in einer Pufferlösung vorgequollener und äquilibrierter schwacher Anionenaustauscher in der Cl⁻-Form, wie DEAE-Sephadex-A 50, verwendet und die Behandlung bei einem ph-Wert von 8 bis 10 durchgeführt. Ein spezielles Beispiel für eine solche Pufferlösung ist 0,01 mol/l Tris-HCl, welche 0,04 mol/l NaCl und 0,001 mol/l Cystein enthält und einen pH-Wert von 8,0 hat.

Bei der Verwendung des Anionenaustauschers wird die Substanzfraktion einer solchen Menge Anionenaustauscher zugesetzt, die zur vollständigen Adsorption der Angiotropine und der positiv adsorbierenden Begleitproteine ausreicht. Üblicherweise genügen dazu zwei Volumenteile gequollener Anionenaustauscher pro Volumen konzentrierter Proteinfraktion. Die Reaktion kann entweder als Chromatographieverfahren oder als leichter handhabbares Eintopfadsorptionsverfahren gestaltet werden. Im Eintopfverfahren wird die überstehende Flüssigkeit mit den negativ adsorbierten Proteinen von dem mit den positiv adsorbierten RNP und andere Substanzen beladenen Anionenaustauscher, beispielsweise durch Filtrieren (in der Chromatographiesäule), Dekantieren oder Zentrifugieren (im Eintopfverfahren) abgetrennt. Der beladene Anionenaustauscher wird durch Waschen mit Wasser oder einer Salzlösung, welche eine zu 0,04 mol/l NaCl äquivalente maximale Ionenstärke hat, vorzugsweise höchstens etwa 15°C von anhaftenden, negativ adsorbierten Verbindungen befreit. Ein spezielles Beispiel für eine zum Auswaschen geeignete Salzlösung ist die erwähnte Tris-Hcl-Pufferlösung vom ph-Wert 8,0.

Der von negativ adsorbierten Verbindungen befreite, mit RNP und anderen Substanzen beladene Anionenaustauscher wird nun mit einer Proteine nicht schädigenden wäßrigen Salzlösung eluiert, welche eine größer als 0,04 mol/l NaCl entsprechende Ionenstärke und einen ph-Wert zwischen 4,0 und 10,0 hat. Vorzugsweise wird eine Salzlösung hoher Ionenstärke mit einem ph-Wert von 5,0 bis 7,0 verwendet. Ein spezielles Beispiel für eine derartige Salzlösung ist eine 2,0 mol/l NaCl-Lösung, welche mit 0,01 mol/l Piperazin-Hcl um ph-Wert 6,5 gepuffert ist und welche 0,001 mol/l Cystein enthält.

Wird die Anionenaustauscherreaktion als Chromatographieverfahren gestaltet, so kann die Elution der RNP und anderen Substanzen auch durch einen linearen NaCl-Konzentrationsgradienten erfolgen.

Als Kationenaustauscher eignen sich für die Reinigung der Proteinfraktion beispielsweise mit Epichlorhydrin vernetzte Dextran-(Sephadex) oder Zellulosematrizen, an welche funktionelle Gruppen mit Kationenaustauscherkapazität gekoppelt sind. Sie können nach Verwendung durch Regeneration wiederholt gebraucht werden. Bevorzugt wird ein schwach saurer Kataionenaustauscher in der Na⁺-Form, wie CM-Sephadex C-50, verwendet, und die Behandlung bei einem ph-Wert von 4 bis 6 durchgeführt. Die Substanzfraktionen können zur Erleichterung der Einstellung der Beladungsgleichgewichte vor der Behandlung mit dem Kationenaustauscher mit einer Proteine nicht schädigenden Salzlösung verdünnt werden, welche eine zu 0,04 mol NaCl/Liter äquivalente maximale Ionenstärke hat. Sie kann gleichzeitig zur Einstellung des ph-Wertes dienen. Ein spezielles Beispiel für eine derartige Salzlösung ist eine 0,001 mol/l Kaliumphosphat-Acetatpufferlösung mit einem Gehalt von 0,04 mol/l NaCl und einem ph-Wert von 4 bis 6. Diese Kationenaustauscherreaktion kann sowohl als Chromatographieverfahren als auch als technisch leicht handhabbares Eintopfverfahren gestattet werden.

Der Kationenaustauscher wird der Substanzfraktion in einer Menge zugesetzt, die ausreicht, um die Proteinfraktion zu adsorbieren. Üblicherweise genügen dazu etwa 2 Volumenteile gequollener Ionenaustauscher pro Volumenteil Proteinfraktion. Sodann wird die überstehende Flüssigkeit von dem mit den Substanzen beladenen Kationenaustauscher, beispielsweise durch Dekantieren oder Zentrifugieren, abgetrennt. Der beladene Kationenaustauscher wird durch Waschen mit Wasser oder einer Salzlösung, welche eine in 0,04 mol/l NaCl äquivalente maximale Ionenstärke hat, vorzugsweise bei einem ph-Wert von etwa 4 bis 6 und einer Temperatur von vorzugsweise höchstens etwa 15 °C von anhaftenden, nicht adsorbierten Verbindung befreit. Ein spezielles Beispiel für eine zum Auswaschen geeignete Salzlösung ist die erwähnte Kaliumphosphat-Acetat-Pufferlösung vom ph-Wert 5,0.

Der von negativ adsorbierten Verbindungen befreite, mit den Substanzen beladene Kationenaustauscher wird nun mit einer Proteine und Nukleinsäuren nicht schädigenden wäßrigen Salzlösung eluiert. Vorzugsweise wird hierzu eine Salzlösung hoher Ionenstärke mit einem ph-Wert von etwa 4 bis 10 verwendet. Spezielle Beispiele für derartige Salzlösungen sind eine wäßrige 0,5 mol/l Kaliumphosphatlösung vom ph-Wert 6,5 bis 7,5 oder eine 2 bis 5 mol/l Natriumchloridlösung vom gleichen ph-Wert.

Für die Chromatographie an Hydroxylapatit werden möglicherweise aus vorangegangenen Schritten vorhandene Salze, z.B. Ammoniumsulfat und vor allen Phosphate, vorzugsweise durch Dialyse oder Ultrafiltration an einer Membran mit einer Auschlußgrenze von 500 Daltons, vor dem Aufbringen auf den Hydroxylapatit entfernt. Abgesehen von der Viskositätserhöhung durch Fremdzusätze ist aber für das Gelingen der Chromatographie an Hydroxylapatit lediglich die Phosphatkonzentration der

Proteinlösung kritisch. Die Eluierung der Substanzen erfolgt durch einen Kaliumphosphat-Konzentrationsgradienten, der vorzugsweise linear ist. Die RNP enthaltenden Fraktionen werden gesammelt und in der nachstehend erwähnten Weise konzentriert.

Die Verwendung von Hydoxylapatit ist für die strukturschonende Reingewinnung der RNP von wesentlicher Bedeutung. Aus technischen und wirtschaftlichen Gründen ist es aber mit erheblichen Schwierigkeiten verbunden, größere Substanzvolumina an Hydroxylapatitsäulen zu chromatographieren. Einerseits neigt nämlich Hydroxylapatit bei größeren Substanzvolumina sehr stark zur Verstopfung und wird dadurch unbrauchbar. Andererseits ist Hydroxylapatit teuer, was seinem Einsatz in größerem Maßstab entgegensteht. Aus diesen Gründen ist es im erfindungsgemäßen Verfahren bevorzugt, aus den Substanzfraktionen, in denen die bioaktiven RNP als Spuren enthalten sind, bereits vor der Chromatographie an Hydroxylapatit einen Großteil der begleitenden Fremdproteine durch geeignete Verfahrensschritte abzutrennen und dadurch das Proteinvolumen, das auf die Hydroxylapatitsäule aufgebracht werden muß, entscheidend zu verkleinern.

Bei der Zonenpräzipitationschromatographie (vgl. J. Porath, Nature, Bd. 196 (1962), S. 47-48) werden Proteinverunreinigungen der bioaktiven RNP durch Aussalzfraktionierung der Proteine mittels eines Salzkonzentrationsgradienten abgetrennt.

Grundprinzip der Proteintrennung mittels der Zonenpräzipitationschromatographie sind unterschiedliche, strukturgegebene reversible Löslichkeitseigenschaften der Proteine. Sie gehören zu den empfindlichsten molekularen Trennparametern und wurden häufig als Kriterium für den Nachweis der molekularen Einheitlichkeit eines Proteins verwendet. Dabei können Temperaturen und ph-Wert, Dimension der Säule, Art des Salzes, Form des Gradienten und Beladung der Säule in einem verhältnismäßig breiten Bereich variiert werden.

Die Temperatur bei der Zonenpräzipitationschromatographie kann etwa 0 bis 40°C betragen. Bevorzugt ist ein Temperaturbereich von etwa 0 bis 10 °C, insbesondere etwa 4 bis 6 °C. Der ph-Wert kann zwischen etwa 4 und 10 liegen; bevorzugt ist ein ph-Wert im Bereich von 6 bis 8, insbesondere etwa 7. Das Verhältnis von Länge: Durchmesser der verwendeten Säule soll größer als etwa 10 : 1 sein, bevorzugt ist ein Verhältnis von 30 bis 100 : 1, insbesondere etwa 50 : 1. Als Salze kommen alle Proteine und Nukleinsäuren nicht schädigenden Salze mit Auswirkung in Frage. Beispiele für solche Salze sind Natriumkaliumphosphat, Ammoniumsulfat und Natriumsulfat. Bevorzugt wird Ammoniumsulfat verwendet.

Der Salzkonzentrationsgradient kann jede beliebige Form haben, so lange die Aussalzpunkte der Proteine laufstreckenmäßig getrennt sind. Bevorzugt sind lineare Konzentrationsgradienten, insbesondere ein ansteigender linearer Konzentrationsgradient von 25 bis 100 % Ammoniumsulfatsättigung. Die Beladung der Säule beträgt höchstens etwa 5 %, vorzugsweise etwa 1 %.

Die Kreislauf- oder Kaskadenmolekularsiebfiltration kann unter den Bedingungen durchgeführt werden, die vorstehend für die anlaytische Molekularsiebfiltration beschrieben sind. Es können die gleichen Molekularsiebe und die gleichen Säulenbedingungen verwendet werden. Bevorzugt ist Sephadex G 50 bei einem Länge-Durchmesser-Verhältnis der Säule von mindestens etwa 50 : 1 und einer Beladung von höchstens etwa 3 % des Säuleninhaltes. Als Lösungsmittel und zur Eluierung werden vorzugsweise die bei der anlaytischen Molekularsiebfiltration benutzten Lösungsmittel eingesetzt.

Bei der Kreislaufmolekularsiebfiltration wird das Eluat bei den festgelegten Separationsgrenzen im Kreislauf wieder in die gleiche Säule geleitet. Auf diese Weise wird die Laufstrecke der Proteine differentiell verlängert. Bei einer anderen Ausführungsform, der Kaskadenmolekularsiebfiltration, wird das Eluat bei den festgelegten Separationsgrenzen auf eine neue Säule mit gleichen oder ähnlich definierten Parametern geleitet.

Zwischen den vorstehend erläuterten Reinigungsschritten können die erhaltenen, bioaktive RNP enthaltenden Substanzlösungen zu nachfolgenden Auftrennungen der Proteine von unerwünschten Salzen gereinigt und konzentriert werden. Diese Konzentration (Abtrennen des Großteils der wäßrigen Salzlösung von den Proteinen) kann auf verschiedene Weise erfolgen. Beispielsweise können die bioaktiven RNP und die Begleitsubstanzen durch Ultrafiltration oder Entwässerungsdialyse an einer Membran mit der Ausschlußgrenze 500 Daltons oder durch Lyophilisieren konzentriert werden. Dazu kann auch eine Molekularsiebfiltration durch Wahl der entsprechenden mobilen Phase in üblicher Weise modifiziert angewendet werden. Bei den Molekularsiebfiltrationen wird der Substanzlösung vorzugsweise etwa 0,4 mol/l Ammoniumsulfat zugesetzt. Im Gegensatz zu höheren Konzentrationen hat das Ammoniumsulfat in dieser Konzentration einen starken Einsalzeffekt gegenüber Proteinen. Durch diese Maßnahmen werden demnach die Proteine während der Molukularsiebfiltration in Lösung gehalten. Ferner verhindert Ammoniumsulfat das bakterielle Wachstum und hemmt gewisse Enzyme. Dadurch trägt es zur Stabilisierung der bioaktiven RNP bei, vor allem wenn die Chromatographie bei höheren Temperaturen (über etwa 20 °) und unter nicht sterilen Bedingungen durchgeführt wird.

Die Temperatur und ph-Bedingungen sind bei der Durchführung der Reinigungsschritte nicht besonders kritisch. Wenn die Erhaltung der nativen Konformation der Substanzen beabsichtigt ist, empfiehlt sich die Einhaltung einer Temperatur im Bereich von etwa 0 bis 8 °C, vorzugsweise etwa 0 bis 4 °C. Ferner müssen die Trenn- und Reinigungsstufen unter im wesentlichen physiologischen Ph- und Salzbedingungen durchgeführt werden. Ein wesentlicher Vorteil des erfindungsgemäßen Verfahrens besteht darin, daß die Einhaltung dieser Bedingungen erstmals leicht möglich ist. Zur Oxidationsverhinderung wird die Substanzlösung vorzugsweise ferner mit etwa 0,001 mol/l Cystein versetzt.

Die erhaltenen bioaktiven RNP können in einer gepufferten physiologischen Salzlösung, beispielsweise in 0,0015 mol/l Natriumkaliumphosphatlösung, die 0,15 mol/ (0,9 %) NaCl und 0,001 mol/l Cystein enthält und einen ph-Wert von 7,4 aufweist, nach üblicher Filtersterilisation (0,2 *µ*m Porenweite) nativ und biologisch aktiv auch bei Raumtemperatur (für mindestens 200 Stunden) oder eingefroren bei -25 °C (für mindestens 5 Jahre) aufbewahrt werden. Diese Stabilität der bioaktiven RNP kann unter anderem als eines der Kriterien für ihren hochreinen Zustand angesehen werden.

Die erfindungsgemäßen RNP können auch unter Verwendung chemisch oder biologisch sythetisierter Teilsequenzen oder Teilen und homologen Sequenzen davon hergestellt werden. Bevorzugt ist, daß man die chemisch oder biologisch synthetisierten Oligonukleotide- oder Antisensenukleotidsequenzen in vivo oder in vitro, welche die nach Anspruch 1 gegebene Sequenzen codieren, mit mindestens 6 Basen in der PCR-Reaktion einsetzt oder die Antisense-Bioprozeßtechnik einsetzt.

Die Beispiele erläutern die Erfindung. In den Beispielen wird die Gewinnung der RNP-Morphogene ausgehend von Leukozyten aus Schweineblut beschrieben. Die Erfindung ist jedoch nicht auf diese Ausführungsform beschränkt. Es können auch Zellen des retikuloendothelialien Systems bzw. Entzündungs-, Wundgewebe-oder flüssigkeit (Exsudat) anderer Säuger verwendet werden.

Die vorliegende Erfindung betrifft ferner die Verwendung der vorstehend beschriebenen RNPs zur Bereitstellung von Arzneimitteln. Diese Arzneimittel enthalten gegebenenfalls zusätzlich einen pharmazeutisch verträglichen Träger. Geeignete Träger und die Formulierung derartiger Arzneimittel sind dem Fachmann bekannt. Zu geeigneten Träger zählen beispielsweise Phosphat-gepufferte Kochsalzlösungen, Wasser, Emulsionen, beispielsweise Öl/Wasser-Emulsionen, Netzmittel, sterile Lösungen etc. Die Verabreichung der Arzneimittel kann oral oder parenteral erfolgen. Zu den Verfahren für die parenterale Verabreichung gehören die topische, intraarterielle (z.B. direkt zu dem Tumor), intramuskuläre, subkutane, intramedulläre, intrathekale, intraventrikuläre, intravenöse, intraperitoneale oder intranasale Verabreichung. Die geeingete Dosierung wird von dem behandelnden Arzt bestimmt und hängt von verschiedenen Faktoren ab, beispielsweise von dem Alter, dem Geschlecht, dem Gewicht des Patienten, der Art der Verabreichung etc. ab. In einer bevorzugten Ausführungsform können die erfindungsgemäßen RNPs einzeln oder als Gemisch lokal bei Säugern, z.B. Menschen, in einer Menge von > 1 fmol gegeben werden. Die Schwellendosis der Wirksamkeit in vivo beträgt > 50 fmol, vorzugsweise 2,5 fmol. Diese Arzneimittel eignen sich zur spezifischen Beeinflussung der Angiomorphogenese und des vaskulären Zustandes eines Gewebes eines Körpers eines Säugers. Diese Arzneimittel können zur Erfüllung der gleichen Aufgaben auch mindestens ein anti-RNP-Immunglobulin und/oder molekularbiologische Äquivalenzstrukturen enthalten. Vorzugsweise wird das entstandene Arzneimittel zur funktionellen Beeinflussung der Angiogenese verwendet.

## Patentansprüche

1. Metallhaltige Ribonukleotidproteine enthaltend ein Protein aus der Familie der S100-Proteine, eine RNA und Kupfer als Metallion in Form eines ternären Komplexes,
**dadurch** gekenzeichnet,
daß die RNA des ternären Komplexes folgende: Sequenzen aufweist:
(a1) (ARNA I) 
Klon-3a (ARNA I)
AAAAAAAAGGUUUUCAUGCGUGCUCACAGAUCAGCUCUUUCUGGAUUGAAAAGCU
AAGCACAGAACAUGGCAAAAUCCUUUCAUAUGGCUGUGUUUACAAACAAAAAGU
AUAAACAUCUUGAGCAAACAGAAAUGGUGAGGAAAACUUUGUUAGCAGAUUAG
 oder
(a2) ARNA VI

Klon-P10 (ARNA VI)
UUACAGCUCUUCUGUUUAUAAGUUAUUCAAUACCAAAUUAGUAGUUUGUAUGUUA
UAAAUUUGUAGGAAAAUAAUUAUAUAUGCUUACUUUGUACAUAAAAAUAAAAACAU
GACUUCUUUAGACACUCCUUCAUUAGAAAUAAAAUAAAAUAAACUAUUAGCAGUUU
GACUUCAUGUUCUGUCUGUAGGUCAUGGAAUCCUGUCCUUACAAUAUUUAUUGAU
UGUGAAAAUAUCAGUAAAUAAGCAAUUGAAUAUGUUUACCUUUUCUUCUAGUCAC
UAUGUUCUUAGAGUUAUGACA

2. Verfahren zur Herstellung eines metallhaltigen nach Anspruch 1,
**dadurch gekennzeichnet, daß** man Laukozyten oder Entzündungsgewebe homogenisiert oder Leukozyten kultiviert und die entstandenen RNPs aus den Homogenaten oder aus den Überständen der Kulturlösung durch Standardmethoden gewinnt.

3. Verwendung der Ribonukleotidproteine nach Anspruch 1 zur Herstellung eines Arzneimittels zur spezifischen Beeinflussung der Angiogenese.

## Claims

1. Metal-containing ribonucleotide proteins containing a protein of the family of S100 proteins, an RNA and copper as metal ion in the form of a ternary complex, **characterised in that** the RNA of the ternary complex is of the following sequences:
(a1) (ARNA I) 
Klon-3a (ARNA I)
AAAAAAAAGGUUUUCAUGCGUGCUCACAGAUCAGCUCUUUCUGGAUUGAAAAGCU
AAGCACAGAACAUGGGAAAAUUCCUUUCAUAUGGCUGUGUUUACAAACAAAAAGU
AUAAACAUCUUGAGCAAACAGAAAUGGUGAGGAAAACUUUGUUAGCAGAUUAG
 or
(a2) ARNA VI

Klon-P10 (ARNA VI)
UUACAGCUCUUCUGUUUAUAAGUUAUUCAAUACCAAAUUAGUAGUUUGUAUGUUA
UAAAUUUGUAGGAAAAUAAUUAUAUGCUUACUUUGUACAUAAAAAUAAAAACAU
GACUUCUUUAGACACUCCUUCAUUAGAAAUAAAAUAAAAUAAACUAUUAGCAGUUU
GACUUCAUGUUCUGUCUGUAGGUCAUGGAAUCCUGUCCUUACAAUAUUUAUUGAU
UGUGAAAAUAUCAGUAAAUAAGCAAUUGAAUAUGUUUACCUUUUCUUCUAGUCAC
UAUGUUCUUAGAGUUAUGACA

2. Method for producing a metal containing ribonucleotide protein according to Claim 1, **characterised in that** leucocytes or inflammation tissue are homogenised or leucocytes are cultivated, and thus acquired RNPs are obtained from the homogenates or from a surplus of the culture solution by means of standard method.

3. Use of ribonucleotide proteins according to Claim 1 for producing a pharmaceutical for specific effect on the angiogenesis.

## Revendications

1. Protéines ribonucléotidiques contenant du métal, comprenant une protéine de la famille des protéines S100, un ARN et du cuivre comme ion métallique, sous la forme d'un complexe ternaire, **caractérisées en ce que** l'ARN du complexe ternaire présente les séquences suivantes :
(a1)(ARNA I) 
Clone-3a (ARNA I)
AAAAAAAAGGUUUUCAUGCGUGCUCACAGAUCAGCUCUUUCUGGAUUGAAAAGCU
AAGCACAGAACAUGGGAAAAUUCCUUUCAUAUGGCUGUGUUUACAAACAAAAAGU
AUAAACAUCUUGAGCAAACAGAAAUGGUGAGGAAAACUUUGUUAGCAGAUUAG
 ou
(a2) ARNA VI

Clone-P10 (ARNA VI)
UUACAGCUCUUCUGUUUAUAAGUUAUUCAAUACCAAAUUAGUAGUUUGUAUGUUA
UAAAUUUGUAGGAAAAUAAUUAUAUAUGCUUACUUUGUACAUAAAAAUAAAAACAU
GACUUCUUUAGACACUCCUUCAUUAGAAAUAAAAUAAAAUAAACUAUUAGCAGUUU
GACUUCAUGUUCUGUCUGUAGGUCAUGGAAUCCUGUCCUUACAAUAUUUAUUGAU
UGUGAAAAUAUCAGUAAAUAAGCAAUUGAAUAUGUUUACCUUUUCUUCUAGUCAC
UAUGUUCUUAGAGUUAUGACA

2. Procédé de fabrication d'une protéine ribonucléotidique contenant du métal selon la revendication 1, **caractérisé en ce que** l'on homogénéise des leucocytes ou du tissu inflammatoire, ou **en ce que** l'on cultive des leucocytes et **en ce que** l'on récupère les PRN obtenues à partir des homogénats ou à partir des surnageants de la solution de culture par des procédés standard.

3. Utilisation des protéines ribonucléotidiques selon la revendication 1 en vue de fabriquer un produit pharmaceutique pour influencer spécifiquement l'angiogenèse.
